Europäisches Patentamt

**European Patent Office**

**Office européen des brevets**

(19)

(11) Veröffentlichungsnummer : **0 008 683**
**B1**

(12)

# EUROPÄISCHE PATENTSCHRIFT

(45) Veröffentlichungstag der Patentschrift :
17.03.82

(51) Int. Cl.³ : **C 07 D251/64**

(21) Anmeldenummer : 79102771.7

(22) Anmeldetag : 02.08.79

(54) **Verfahren zur Herstellung weitgehend monomerer methylverätherter Methylolmelamine.**

(30) Priorität : 13.09.78 DE 2839713.

(43) Veröffentlichungstag der Anmeldung :
19.03.80 (Patentblatt 80/06)

(45) Bekanntmachung des Hinweises auf die Patenterteilung : 17.03.82 Patentblatt 82/11

(84) Benannte Vertragsstaaten :
**AT CH DE FR GB IT NL SE**

(56) Entgegenhaltungen :
**CH - A - 432 839**
**FR - A - 1 086 826**
**FR - A - 2 386 531**
**GB - A - 611 013**
**US - A - 2 998 410**

(73) Patentinhaber : **CASSELLA Aktiengesellschaft**
**Hanauer Landstrasse 526**
**D-6000 Frankfurt am Main 61 (DE)**

(72) Erfinder : **Hönel, Hans, Dr.**
**Meerholzer Strasse 42**
**D-6000 Frankfurt am Main 61 (DE)**
Erfinder : **Keller, Karlfried, Dr.**
**Bischofsheimer Strasse 21**
**D-6000 Bergen-Enkheim 60 (DE)**
Erfinder : **Michel, Walter, Dr.**
**Fuldaer Strasse 27**
**D-6000 Frankfurt am Main 61 (DE)**
Erfinder : **Schön, Manfred, Dr.**
**Saalburgring 3**
**D-6054 Rodgau 2 (DE)**

(74) Vertreter : **Urbach, Hans-Georg, Dr. et al**
**Hanauer Landstrasse 526**
**D-6000 Frankfurt am Main 61 (DE)**

Imprimerie Jouve, 18, rue St-Denis, 75001 Paris, France

# Verfahren zur Herstellung weitgehend monomerer methylverätherter Methylolmelamine

Die Erfindung betrifft ein Verfahren zur Herstellung weitgehend monomerer methylverätherter Methylolmelamine, die pro Mol Melamin mehr als 5 Mole Formaldehyd und mehr als 4 Mole methylverätherter Methylolgruppen enthalten und bei einem Festkörpergehalt von mindestens 95 Gew.% bei 25 °C eine Viskosität von weniger als 20 Pa·s (Pa·s = Pascalsekunde ; SI-Einheit der dynamischen Viskosität) 'besitzen.

Derartige Melaminharze werden z.B. als sogenannte High-Solid-Lack-Harze zur Herstellung von Lacken verwendet. Wegen ihrer geringen Viskosität bei gleichzeitigen relativ hohen Körpergehalten brauchen die Lacke bei Verarbeitungs-bedingungen nur wenig flüchtige Lösungsmittel zu enthalten. Dadurch werden bei der Verarbeitung der Lacke, z.B. beim Einbrennen aufgespritzter Einbrennlacke, auch nur geringe Mengen flüchtiger organischer Lösungsmittel frei, was zum Schutze der Umwelt sehr erwünscht ist.

Nach Beispiel 1 der britischen Patentschrift 1 030 268 können derartige Melaminharze durch Methylolierung von Melamin mit 11 Molen Paraformaldehyd pro Mol Melamin in methanolischer Lösung in Gegenwart eines basischen Katalysators sowie anschließender zweimaliger Verätherung mit überschüssigem Methanol in Gegenwart einer starken anorganischen Säure hergestellt werden. Da pro Mol Melamin höchstens 6 Mole Formaldehyd gebunden werden, tritt durch den im Überschuß vorhandenen Formaldehyd entweder eine starke Belastung von Abwasser und Abluft auf, oder der im Überschuß vorhandene Formaldehyd muß mit zusätzlichen Kosten aufgearbeitet werden. Setzt man bei diesem Verfahren anstelle von 11 Molen Paraformaldehyd 6,5-8 Mole Paraformaldehyd pro Mol Melamin ein, so erhält man Produkte, die infolge eines höheren Kondensations- und niedrigeren Verätherungsgrads höher viskos sind und bei einem Festkörpergehalt von 95 Gew.% eine Viskosität von mehr als 1 000 Pa·s besitzen. Außerdem besitzen die so hergestellten Melaminharze nur eine geringere Haltbarkeit sowie eine verminderte Verträglichkeit mit hydrophoben Komponenten und sind infolge dessen als sogenannte High-Solid-Lack-Harze wenig geeignet.

In der US-Patentschrift 2 918 452 wird ein Verfahren zur Herstellung weitgehend monomerer methylverätherter Melaminharze beschrieben, wobei zuerst in einer Suspension eines Kohlenwasserstoffs, wie Xylol, Melamin mit Paraformaldehyd im Molverhältnis 1 : 6,5 bis 20 in Gegenwart von 8 bis 20 % Wasser bei einem pH-Wert von 8 bis 13 methyloliert wird. Das Methylolierungsprodukt erstarrt zu einer festen Masse, die gemahlen wird und eine Suspension des Methylolmelamins in dem Kohlenwasserstoff liefert. Die anschließende saure Verätherung mit überschüssigem Methanol kann in Gegenwart des Kohlenwasserstoffs oder nach seiner Entfernung durchgeführt werden. Als Nachteile dieses Verfahrens sind neben der Verwendung von Paraformaldehyd, der in technisch aufwendiger Weise durch Einengen von wäßrigen Formaldehydlösungen erhalten wird, besonders die Zerkleinerung des als Zwischenprodukt anfallenden, kristallinen Methylolmelamins und die Verwendung des feuergefährlichen, die Umwelt belastenden Kohlenwasserstoffs zu erwähnen, dessen Verwendung außerdem die Raum-Zeit-Ausbeute herabsetzt.

In Beispiel 1 der US-Patentschrift 3 020 255 wird zur Herstellung eines methylverätherten Methylolmelamins Melamin mit 6,18 Molen 37 %igem wäßrigen Formaldehyd gemischt und der pH-Wert des Systems mit Triäthylamin auf 7,2 bis 7,8 eingestellt. Innerhalb von 10 Minuten wird auf 90 °C erhitzt und danach abkühlen gelassen. Der dabei erhaltene Niederschlag wird bei 40 bis 50 °C im Vakuum bis auf einen Wassergehalt von 6 % entwässert und anschließend mit 4,7 Molen Methanol sauer 10 Minuten lang bei Raumtemperatur veräthert. Nachteilig ist die erforderliche Entwässerung, bei deren Verlauf das Produkt nicht mehr rührbar wird. Außerdem werden ohne mehrmalige Umkristallisationen nur hochviskose methylverätherte Methylolmelamine als Endprodukte erhalten.

Bei dem aus der US-Patentschrift 2 998 410 bekannten Verfahren muß das zunächst hergestellte hochmethylolierte Melamin isoliert und auf Wassergehalte von weniger als 20 % getrocknet werden, bevor es in einer nachfolgenden Stufe veräthert werden kann. Dabei muß das Methylolmelamin nach der Methylolierung mit zusätzlichem Wasser versetzt werden, damit es auf üblichen Vorrichtungen abgetrennt werden kann. Das Verfahren ist kostenungünstig und seine Raumzeitausbeute schlecht.

Bei dem Verfahren der US-Patentschrift 2 998 411 wird während der Methylolierung durch laufende Zugabe basischen Katalysators der pH-Wert auf 8 bis 11 gesteigert. Das erhaltene Methylolmelamin muß abgetrennt und, falls es einstufig veräthert werden soll, getrocknet werden. Ohne Trocknung des Methylolmelamins muß eine zweistufige Verätherung durchgeführt werden, wobei in der ersten Verätherungsstufe normalerweise die Anwendung eines Schleppmittels, wie Xylol oder Toluol, erforderlich ist.

Bei dem aus der GB-Patentschrift 674 948 bekannten Verfahren zur Herstellung weitgehend monomerer methylverätherter Methylolmelamine wird Melamin mit 6 bis 9 Molen wäßrigem Formaldehyd bei 50 bis 80 °C in möglichst kurzer Zeit, beispielsweise 40 Minuten, methyloliert, und der Reaktionsansatz wird anschließend ohne Isolierung des Methylolmelamins in Gegenwart einer starken Säure mit Methanol veräthert. Die nach diesem Verfahren hergestellten Melaminharze sind jedoch bei 25 °C und einem Festkörpergehalt von 95 Gew.% hochviskos und besitzen eine Viskosität von mehr als 1 000 Pa·s.

Die bisher bekanntgewordenen Verfahren zur Herstellung von weitgehend monomeren methylverätherten Methylolmelaminen erfordern entweder eine aufwendige Zwischenisolierung bzw. mindestens teilweise Entwässerung des Methylolmelamins, oder sie liefern, wenn sie ohne Zwischenisolierung des Methylolmelamins arbeiten, nur hochviskose methylverätherte Methylolmelamine. Die Schwierigkeiten der bisherigen Verfahren sind darauf zurückzuführen, daß das bei der Methylolierung ausfallende hochmethylolierte Melamin den Ansatz immer schwieriger rührbar macht, so daß eine Verdünnung der Suspension und anschließende Isolierung des Methylolmelamins erforderlich wird. Wenn man, um einen noch rührbaren Ansatz zu erhalten, auf eine vollständige Methylolierung verzichtet, dann erhält man als Endprodukte hochviskose methylverätherte Methylolmelamine, weil die freien Aminogruppen der nicht vollständig methylolierten Methylolmelamine bevorzugt mit Methylolgruppen zu höher molekularen Harzanteilen mit entsprechend hoher Viskosität kondensieren.

Es ist auch bereits ein Verfahren zur Herstellung niedrigviskoser, weitgehend monomerer, methylverätherter Methylolmelamine vorgeschlagen worden (deutsche Patentanmeldung P 27 16 006,1), bei dem Melamin und Formaldehyd im Molverhältnis 1 : 6,2 bis 1 : 8 in Gegenwart eines basischen Katalysators in einer Reaktionsmischung, die 10 bis 40 Gew.% Wasser und 40 bis 20 Gew.% Methanol enthält, bei Temperaturen von 40 bis 60 °C zu einer Suspension von Methylolmelamin umgesetzt und anschließend in Gegenwart von insgesamt 15 bis 30 Molen Methanol pro Mol Melamin bei Temperaturen von 25 bis 50 °C in Gegenwart einer starken Säure veräthert wird. Bei diesem Verfahren können bei größeren Ansätzen wegen der verhältnismäßig niedrigen Temperatur bei der Methylolierung Probleme bei der Abführung der Reaktionswärme auftreten.

Die schweizerische Patentschrift 432 839 betrifft ein Verfahren zur Herstellung mit Wasser mischbarer Methylolmelamin-alkylätherharze, bei dem man Melamin und Formaldehyd im Molverhältnis 1 : (2 bis 10) bei pH 7 bis 10 und ca. 90 °C mindestens 30 Minuten lang und höchstens so lange vorkondensiert, bis 1 Teil der Harzlösung mit 2 Teilen Wasser von 20° eine Trübung ergibt und sodann unter Zugabe von 0,8 bis 1,5 Mol Alkohol mit 1 oder 2 C-Atomen — pro Mol in der Harzlösung vorhandenen Wassers in Gegenwart von 0,001 bis 0,02 Äquivalenten, bezogen auf Melamin — einer mittelstarken bis starken Säure bei bis zum Siedepunkt reichender Temperatur veräthert. Die erhaltenen Harze werden auf einen Festkörpergehalt von 60 Gew.% eingeengt. Bei stärkerer Einengung werden nicht mehr fließfähige Produkte erhalten.

Die französische Patentschrift 1 086 826 betrifft ein Verfahren zur Herstellung von mit Methanol verätherten methylolierten Aminotriazinen, die in Wasser unlöslich oder nur begrenzt löslich sind.

Bei diesem Verfahren wird ein Aminotriazin, z.B. Melamin, in wäßriger Lösung mit Formaldehyd bei 90 bis 95 °C vorkondensiert und die erhaltene Lösung anschließend mit Methanol bei Rückflußtemperatur veräthert. Nach den Beispielen beträgt das Molverhältnis Melamin : Formaldehyd = 1 : (3 bis 6). Auch nach diesem Verfahren können keine niedrigviskosen Harze hergestellt werden.

Der Erfindung lag daher die Aufgabe zugrunde, die Nachteile der bisherigen Verfahren zur Herstellung weitgehend monomerer methylverätherter Methylolmelamine, die pro Mol Melamin mehr als 5 Mole Formaldehyd und mehr als 4 Mole methylverätherter Methylolgruppen enthalten und bei einem Festkörpergehalt von mindestens 95 Gew.% bei 25 °C eine Viskosität von weniger als 20 Pa·s besitzen, zu vermeiden. Insbesondere sollte bei dem erfindungsgemäßen Verfahren bei der Methylolierung eine gut rührbare Suspension von hochmethylolierten Methylolmelaminen erhalten werden, die ohne Isolierung oder Trocknung der Methylolmelamine direkt anschließend im selben Gefäß einstufig und ohne Anwendung eines Schleppmittels veräthert werden kann.

Erfindungsgemäß wird die Aufgabe dadurch gelöst, daß Melamin mit einer wäßrigen, zwischen 30 und 50 Gew.% Formaldehyd enthaltenden Formalinlösung, die auf pH-Werte von 6,7 bis 7,3 eingestellt wurde, im Molverhältnis 1 : 6,5 bis 1 : 8 während 1 1/2 bis 5 Stunden bei 50 bis 85 °C methyloliert wird und die erhaltene, gut rührbare Suspension des Methylolmelamins mit 15 bis 30 Molen Methanol pro Mol Melamin bei Temperaturen von 30 bis 60 °C in Gegenwart einer starken Säure veräthert wird.

Das erfindungsgemäße Verfahren eignet sich vorzugsweise zur Herstellung von solchen weitgehend monomeren methylverätherten Methylolmelaminen, die pro Mol Melamin mehr als 5,5 Mole Formaldehyd und 4,3 bis 5,1 Mole methylverätherter Methylolgruppen enthalten.

Bei dem erfindungsgemäßen Verfahren wird Formaldehyd in Form der üblichen, zwischen 30 und 50 Gew.% Formaldehyd enthaltenden, wäßrigen Formalinlösungen eingesetzt, die zur Stabilisierung bis zu 12 Gew.% Methanol enthalten können. Je geringer der Methanolgehalt der Formalinlösung ist, um so niedrigviskosere Endprodukte werden normalerweise erhalten. Gegebenenfalls können auch Mischungen von niedriger prozentigen wäßrigen Formaldehydlösungen und Paraformaldehyd verwendet werden. Pro Mol Melamin müssen 6,5 bis 8 Mole, vorzugsweise 7 bis 7,5 Mole Formaldehyd vorhanden sein. Formaldehydlösungen, die mehr als 50 Gew.% Formaldehyd enthalten, werden wegen ihrer geringen Stabilität nur für spezielle Anwendungen eingesetzt. Die mit ihnen nach dem erfindungsgemäßen Verfahren hergestellten Methylolmelamine neigen insbesondere bei größeren Ansätzen wegen des als Verdünnungsmittel fehlenden Wassers zur Bildung von Krusten an der Gefäßwandung.

Erfindungsgemäß wird die wäßrige Formalinlösung vor ihrer Vereinigung mit dem Melamin auf pH-Werte von 6,7 bis 7,3 eingestellt. Für diese pH-Einstellung können basisch reagierende organische und/oder anorganische Verbindungen verwendet werden, wie z.B. Alkalihydroxide, wie Natrium- oder Kaliumhydroxid, Alkalicarbonate oder Alkalihydrogencarbonate, wie z.B. Natrium- oder Kaliumcarbonat, Natrium- oder Kaliumhydrogencarbonat, sekundäre Alkaliphosphate, wie z.B. Dinatriumhydrogenphosphat, Alkaliborate, organische Amine, wie Trialkylamino, z.B. Triäthylamin, Tripropylamin und Tributylamin. Für die pH-Einstellung der Formaldehydlösung wird vorzugsweise Borax verwendet, weil hierbei besonders niedrig viskose und damit besonders gut rührbare Suspensionen hochmethylolierter Methylolmelamine erhalten werden.

Zur Einstellung des pH-Wertes der Formaldehydlösung auf Werte von 6,7 bis 7,3 sind normalerweise 0,01 bis 1 Gew.% der basisch reagierenden Verbindung, bezogen auf das Gewicht des in der Formaldehydlösung vorhandenen Formaldehyds, erforderlich. Die erforderliche Menge der basischen Verbindung ist selbstverständlich vom Säuregehalt der Formaldehydlösung abhängig. Die verwendete Formaldehydlösung sollte möglichst wenig Ameisensäure, und zwar in der Regel weniger als 0,1 Gew.% Ameisensäure, enthalten. Formaldehydlösungen mit einem Gehalt von weniger als 0,01 Gew.% Ameisensäure (hierbei zeigt die Formalinlösung etwa einen pH-Wert von 3,4 bis 3,5) geben besonders gute Ergebnisse. Zur Einstellung des pH-Wertes kann auch ein Gemisch verschiedener basisch reagierender Substanzen benutzt werden. Die auf pH-Werte von 6,7 bis 7,3 eingestellte wäßrige Formaldehydlösung wird mit dem Melamin im Molverhältnis Melamin : Formaldehyd = 1 : (6,5 bis 8) gemischt und unter Rühren auf eine Temperatur von 50 bis 80 °C erwärmt, wobei das Melamin in Lösung geht.

Die Methylolierung wird bei Temperaturen von 50 bis 85 °C, vorzugsweise 65 bis 75 °C, innerhalb von 5 bis 1 1/2 Stunden, vorzugsweise 3 bis 2 Stunden, durchgeführt, wobei der Reaktionsansatz gerührt wird. Da die Methylolierung bei niedriger Temperatur eine längere Reaktionszeit erfordert und umgekehrt der gewünschte Methylolierungsgrad bei hoher Reaktionstemperatur bereits nach kürzerer Zeit erreicht wird, muß der Endpunkt der Methylolierung durch Vorversuche ermittelt werden. Dabei kann davon ausgegangen werden, daß bei gleichen Molverhältnissen Melamin : Formaldehyd die Viskosität der weitgehend monomeren methylverätherten Methylolmelamine sinkt, wenn die Viskosität der wäßrigen Suspension der Methylolmelamine steigt. Da die wäßrige Suspension der Methylolmelamine im Verlaufe der Methylolierung allmählich viskoser wird, muß der Endpunkt der Methylolierung so gewählt werden, daß das Methylolmelamin ohne Rand- und Krustenbildung anfällt und während der Verätherung vollständig in Lösung geht.

Zum Umrühren des Reaktionsansatzes wird zweckmäßigerweise ein randnaher Rührer verwendet, d.h. ein solcher Rührer, der auch in der Nähe der gesamten, von Flüssigkeit bedeckten Behälterwand vorbeirührt, weil dadurch die Bildung von Krusten an der Behälterwand vermieden wird. Derartige Krusten lassen sich, wenn sie sich erst einmal gebildet haben, anschließend nur schwierig oder gar nicht mit Methanol veräthern. Geeignete Rührer sind beispielsweise Anker- und Hakenrührer, die in ihrer Form der Form des Behälters angepaßt worden sind.

Nach dem erfindungsgemäßen Verfahren erhält man eine gut rührbare Suspension von hochmethyloliertem Melamin, die anschließend im gleichen Reaktionsgefäß mit Methanol veräthert werden kann, ohne daß eine Isolierung des Methylolmelamins oder eine Zwischentrocknung erforderlich ist.

Zur Verätherung wird der erhaltenen Suspension Methanol in einer solchen Menge zugefügt, daß pro Mol Melamin insgesamt 15 bis 30 Mole Methanol vorhanden sind. Die Verätherung wird unter Rühren bei Temperaturen von 30 bis 60 °C, vorzugsweise 35 bis 45 °C, in Gegenwart von 0,5 bis 10 Gew.%, vorzugsweise 1 bis 5 Gew.%, einer starken Säure, bezogen auf das Gewicht der Ausgangskomponenten Melamin und Formaldehyd, durchgeführt. Sofern die Verätherung mit Methanol bei einer tieferen Temperatur als die Methylolierung durchgeführt wird, ist es zweckmäßig, die Suspension des Methylolmelamins erst nach dem Zusatz des Methanols auf die Temperatur der Verätherung abzukühlen, weil dadurch die Rührbarkeit des Ansatzes auch unter weniger günstigen Umständen gewährleistet bleibt.

Geeignete starke Säuren können Mineralsäuren, wie z.B. Schwefelsäure, Salzsäure, Phosphorsäure, Carbonsäuren, wie z.B. Trichloressigsäure, oder Sulfonsäuren, wie z.B. p-Toluolsulfonsäure, sein. Auch Gemische starker Säuren können verwendet werden. Vorzugsweise wird Salpetersäure verwendet. Je nach den Reaktionsbedingungen ist die Verätherung nach 20 Minuten bis maximal 3 Stunden beendet. Nach beendeter Verätherung ist eine klare Lösung entstanden. Abschließend wird der Ansatz in bekannter Weise aufgearbeitet, d.h. z.B. mit Natrium- oder Kaliumcarbonat, Natrium- oder Kaliumhydroxid, neutralisiert bzw. schwach alkalisch gestellt und das überschüssige Methanol, zusammen mit dem im Ansatz enthaltenen Wasser, unter vermindertem Druck abdestilliert. Nach vollständiger Entfernung der flüchtigen Anteile wird das Melaminharz zur Abtennung der Salze, beispielsweise über Kieselgur, filtriert.

Die nach dem erfindungsgemäßen Verfahren hergestellten weitgehend monomeren, methylverätherten Methylolmelamine besitzen bei einem Festkörpergehalt von mindestens 95 Gew.% bei 25 °C eine Viskosität von weniger als 20 Pa·s. Derartig niedrig viskose Melaminharze werden jedoch nur dann erhalten, wenn pro Mol Melamin mindestens 6,5 Mole Formaldehyd eingesetzt werden, dessen pH-Wert auf 6,7 bis 7,3 eingestellt worden ist. Beim Einsatz von weniger als 6,5 Mole Formaldehyd pro Mol Melamin entstehen Methylolmelamine, die zu Rand- und Krustenbildung neigen und außerdem infolge von Kondensation unvollständig methylolierter Zwischenprodukte höher viskose Melaminharze ergeben. Besonders umweltfreundliche, niedrigviskose methyloverätherte Methylolmelamine mit nur geringer Belastung von Abwasser und Abluft entstehen bei einem Molverhältnis von Melamin : Formaldehyd = 1 : (7 bis 7,5). Die Verwendung von mehr als 8 Molen Formaldehyd pro Mol Melamin liefert Produkte, die gegenüber den innerhalb der beanspruchten Molverhältnisse hergestellten Produkten keine verbesserten Eigenschaften aufweisen, durch die unnötig hohe Formaldehydmenge jedoch die Umwelt belastet. Außerdem wird durch den höheren Wassergehalt die nachfolgende Verätherung erschwert.

Wenn die wäßrige Formaldehydlösung vor der Melaminzugabe auf pH-Werte unterhalb von 6,7 eingestellt wird, dann entstehen durch Kondensation höhermolekulare Methylolmelamine, die nach der Verätherung höherviskose methylverätherte Methylolmelamine ergeben ; pH-Werte der Formaldehydlösung oberhalb von 7,3 führen bereits nach kurzer Zeit zu hochviskosen klumpigen Methylolmelaminen, die schwer rührbar sind und sich infolge Rand- und Krustenbildung nur teilweise veräthern lassen. Eine Durchführung der Methylolierung unterhalb von 50 °C dauert sehr lange und liefert eine viskose Methylolmelaminsuspension, die nur schlecht oder gar nicht mehr rührbar ist. Eine Methylolierung oberhalb von 85 °C liefert zwar eine dünnflüssige Methylolmelaminsuspension, die jedoch bei der anschließenden Verätherung nur viskose Endprodukte ergibt. Nach der Zugabe des Melamins zu der Formaldehydlösung ändert sich der pH-Wert des Reaktionsansatzes. Eine Verfolgung bzw. Einstellung des sich während der Methylolierung ändernden pH-Wertes ist bei dem erfindungsgemäßen Verfahren nicht erforderlich.

Die bei dem erfindungsgemäßen Verfahren als Zwischenprodukt anfallenden Methylolmelamine und die methylverätherten Methylolmelamine sind, wie in der Melaminharzchemie üblich, Gemische verschiedener chemischer Individuen. Die jeweils angegebene Zusammensetzung stellt die Durchschnittszusammensetzung des Gemisches dar.

Das erfindungsgemäße Verfahren stellt eine einfaches umweltfreundliches und wirtschaftliches Eintopfverfahren dar, das niedrigviskose, weitgehend monomere methylverätherte Methylolmelamine ohne den Einsatz eines großen Überschusses an Formaldehyd, ohne Zugabe eines inerten Lösungsmittels und ohne Isolierung und ohne Zwischentrocknung wasserhaltiger Methylolmelamine in einstufiger Verätherung ohne die Anwendung eines Schleppmittels liefert.

Die nach dem erfindungsgemäßen Verfahren erhaltenen Produkte können z.B. verwendet werden für die Veredlung von Textilien und Papier sowie für den High-Solid-Bereich der Lackierung in Kombination mit Alkydharzen, ölfreien Polyestern oder Acrylatharzen zur Herstellung von hochglänzenden kratzfesten Überzügen mit sehr guter Wetterbeständigkeit, sowie als Haftmittel. Falls bei der Anwendung ein Zusatz von Lösungsmitteln erforderlich ist, werden wegen der geringen Viskosität der Produkte nur geringe Zusatzmengen benötigt.

Die folgenden Beispiele dienen der weiteren Erläuterung der Erfindung. Prozentangaben stellen Angaben in Gewichtsprozenten dar.

Beispiel 1

Molverhältnis Melamin : Formaldehyd : Methanol = 1 : 7,4 : 25

In einem 4-Liter-Dreihalskolben werden 1 139 g einer 39 %igen wässrigen Formaldehydlösung (pH 3,5) durch den Zusatz von 1 g Borax ($Na_2B_4O_7.10H_2O$) auf pH 7 eingestellt. Nach Zugabe von 252 g Melamin entsteht beim Erwärmen des Ansatzes auf 65 °C unter Rühren mit einem Hakenrührer eine klare Lösung. Anschließend wird 2 1/2 Stunden bei 65 °C methyloliert, wobei ein gut rührbarer Brei entsteht. Nach Zugabe von 1 600 g Methanol wird der Kolbeninhalt innerhalb einer Stunde auf 40 °C abgekühlt und zur Verätherung mit 27 g 55 %iger Salpetersäure versetzt. Bei abgeschalteter Heizung und Kühlung ist nach ca. 1 Stunde eine klare Lösung entstanden, die sofort mit Natronlauge auf pH 8,5 eingestellt und unter Wasserstrahlvakuum bis zur Innentemperatur von 85 °C eingeengt wird.

Nach der Filtration über Kieselgur erhält man ein 95 %iges methylverätchertes Methylolmelamin (der Festkörpergehalt wird aus dem Gewichtsverlust berechnet, den 2 g des Produktes beim einstündigen Erhitzen auf 120 °C in einer Aluminiumschale erleiden), das bei 25 °C eine Viskosität von 5,1 Pa·s besitzt und pro Mol Melamin ca. 5,8 Mole Formaldehyd und ca. 4,9 Mole methylverätherter Methylolgruppen enthält.

Wenn der zur Methylolierung verwendete Formaldehyd mit 0,12 g Natriumhydroxid anstelle von 1 g Borax auf pH 7 eingestellt wird, so entsteht ein methylverätchertes Methylolmelamin gleicher Zusammensetzung, das bei 95 % Festkörpergehalt eine Viskosität von 6,7 Pa·s besitzt.

Wird die Methylolierung im obigen Beispiel mit einem Formaldehyd durchgeführt, der mit 0,44 g Kaliumcarbonat anstelle von 1 g Borax auf pH 7 eingestellt wurde, so erhält man nach dem Aufarbeiten ein methylverätchertes Methylolmelamin gleicher Zusammensetzung, das bei 95 % Festkörpergehalt eine Viskosität von 5,9 Pa·s besitzt.

Bei der Methylolierung nach Beispiel 1 mit einem Formaldehyd, der mit 0,58 g Natriumhydrogencarbonat anstelle von 1 g Borax auf pH 7 eingestellt wurde, entsteht nach der Verätherung ein methylverätchertes Methylolmelamin gleicher Zusammensetzung, das bei 95 % Festkörpergehalt eine Viskosität von 4,4 Pa·s besitzt.

Beispiel 2

Molverhältnis Melamin : Formaldehyd : Methanol = 1 : 7,4 : 21

Die nach Beispiel 1 erhaltene wässrige Suspension des Methylolmelamins wird bei 65 °C mit 1 344 g Methanol versetzt und, wie dort beschrieben, nach dem Abkühlen auf 40 °C in Gegenwart von 27 g 55 %iger Salpetersäure veräthert und weiterverarbeitet.

Das erhaltene 95 %ige methylverätherte Methylolmelamin besitzt bei 25 °C eine Viskosität von 9,3 Pa·s und enthält pro Mol Melamin ca. 5,7 Mole Formaldehyd und ca. 4,6 Mole methylverätherte Methylolgruppen.

## Beispiel 3

Molverhältnis Melamin : Formaldehyd : Methanol = 1 : 6,8 : 25

In einem 4-Liter-Dreihalskolben werden 1 046 g einer 39 %igen wässrigen Formaldehydlösung (pH 3,7) durch den Zusatz von 1 g Borax auf pH 7,1 eingestellet. Nach Zugabe von 252 g Melamin erhält man beim Erwärmen auf 75 °C unter Rühren mit einem Hakenrührer eine klare Lösung. Anschließend wird 2 Stunden bei 75 °C methyloliert, wobei ein gut rührbarer Brei entsteht. Nach Zugabe von 1 600 g Methanol wird der Kolbeninhalt innerhalb einer Stunde auf 40 °C abgekühlt und zur Verätherung mit 27 g 55 %iger Salpetersäure versetzt. Die nach ca. 2stündiger Verätherung entstandene klare Lösung wird wie in Beispiel 1 weiterverarbeitet.

Das erhaltene 95 %ige methylverätherte Methylolmelamin besitzt bei 25 °C eine Viskosität von 3,9 Pa·s und enthält pro Mol Melamin ca. 5,7 Mole Formaldehyd und ca. 4,7 Mole methylverätherte Methylolgruppen.

## Beispiel 4

Molverhältnis Melamin : Formaldehyd : Methanol = 1 : 8 : 25

In einem 4-Liter-Dreihalskolben werden 1 231 g einer 39 %igen wässrigen Formaldehydlösung (pH 3,7) durch den Zusatz von 1 g Borax auf pH 7 eingestellt. Nach Zugabe von 252 g Melamin wird der Kolbeninhalt, wie in Beispiel 3 beschrieben, 2 Stunden bei 75 °C gerührt und anschließend, wie dort beschrieben, veräthert und aufgearbeitet. Das erhaltene 95 %ige methylverätherte Methylolmelamin besitzt bei 25 °C eine Viskosität von 5,6 Pa·s und enthält pro Mol Melamin ca. 5,9 Mole Formaldehyd und ca. 4,6 Mole methylverätherte Methylolgruppen.

## Beispiel 5

Molverhältnis Melamin : Formaldehyd : Methanol = 1 : 7,4 : 25

In einem 4-Liter-Dreihalskolben werden 1 139 g einer 39 %igen wässrigen Formaldehydlösung (pH 3,5) durch den Zusatz von 0,6 g Borax auf pH 6,9 eingestellt. Nach Zugabe von 252 Gew.Teilen Melamin wird der Kolbeninhalt unter Rühren mit einem Hakenrührer auf 75 °C erwärmt, wobei eine klare Lösung entsteht. Nach 2 1/2 stündiger

Methylolierung bei 75 °C wird der Kolbeninhalt mit 1 600 g Methanol versetzt und weiterverarbeitet, wie in Beispiel 1.

Das erhaltene 95 %ige Methylverätherte Methylolmelamin besitzt bei 25 °C eine Viskosität von 9,5 Pa·s und enthält pro Mol Melamin ca. 5,6 Mole Formaldehyd und ca. 4,5 Mole methylverätherte Methylolgruppen.

## Beispiel 6

Molverhältnis Melamin : Formaldehyd : Methanol = 1 : 7,4 : 25

In einem 4-Liter-Dreihalskolben werden 1 139 g einer 39 %igen wässrigen Formaldehydlösung (pH 3,9), die zur Stabilisierung 80 g Methanol enthält, durch den Zusatz von 1,1 g Borax auf pH 7,2 eingestellt. Nach Zugabe von 252 g Melamin wird der Kolbeninhalt unter Rühren mit einem Hakenrührer auf 75 °C erwärmt, wobei eine klare Lösung entsteht. Nach 2 1/2 stündiger Methylolierung bei 75 °C erhält man einen gut rührbaren Brei, der nach Zugabe von 1 520 g Methanol weiterverarbeitet wird, wie in Beispiel 1 beschrieben.

Als Endprodukt wird ein 95 %iges methylveräthertes Methylolmelamin erhalten, das bei 25 °C eine Viskosität von 8,3 Pa·s besitzt und pro Mol Melamin ca. 5,8 Mole Formaldehyd und ca. 4,7 Mole methylverätherte Methylolgruppen enthält.

## Beispiel 7

Molverhältnis Melamin : Formaldehyd : Methanol = 1 : 7,4 : 25

In einem 4-Liter-Dreihalskolben werden 987 g einer 45 %igen wässrigen Formaldehydlösung (pH 3,5) durch Zusatz von 1 g Borax auf pH 7,1 eingestellt. Nach Zugabe von 252 g Melamin entsteht beim Erwärmen auf 75 °C unter Rühren mit einem Hakenrührer zunächst eine klare Lösung, nach 2stündiger Methylolierung bei 75 °C ein gut rührbarer Brei des Methylolmelamins, der nach Zugabe von 1 600 g Methanol weiterverarbeitet wird, wie in Beispiel 1 beschrieben.

Das erhaltene 95 %ige methylverätherte Methylolmelamin besitzt bei 25 °C eine Viskosität von 2,3 Pa·s und enthält pro Mol Melamin ca. 5,9 Mole Formaldehyd und ca. 4,9 Mole methylverätherte Methylolgruppen.

## Beispiel 8

Molverhältnis Melamin : Formaldehyd : Methanol = 1 : 7,4 : 25

In einem 4-Liter-Dreihalskolben werden 1 269 g einer 35 %igen wässrigen Formaldehydlösung (pH 3,7) durch Zusatz von 1,8 g Borax auf pH 7,3 eingestellt. Nach Zugabe von 252 g Melamin wird unter Rühren mit einem Hakenrührer 2 Stunden bei 75 °C methyloliert. Der erhaltene, gut rührbare Brei des Methylolmelamins wird nach Zusatz von 1 600 g Methanol weiterverarbeitet, wie in Beispiel 1 beschrieben.

Als Endprodukt wird ein 95 %iges methylveräthertes Methylolmelamin erhalten, das bei 25 °C eine Viskosität von 7,7 Pa·s besitzt und pro Mol Melamin ca. 5,7 Mole Formaldehyd und ca. 4,5 Mole methylverätherte Methylolgruppen enthält.

Beispiel 9

Molverhältnis Melamin : Formaldehyd : Methanol = 1 : 7,4 : 21

In einem 4-Liter-Dreihalskolben werden 1 139 g einer 39 %igen wäßrigen Formaldehydlösung (pH 3,1) durch den Zusatz von 1 g Borax auf pH 6,7 eingestellt. Nach Zugabe von 252 g Melamin wird der Kolbeninhalt unter Rühren mit einem Hakenrührer auf 75 °C erwärmt, wobei eine klare Lösung entsteht. Nach 2stündiger Methylolierung bei 75 °C wird der Kolbeninhalt mit 1 344 g Methanol versetzt und weiterverarbeitet, wie in Beispiel 1.

Das erhaltene 95 %ige methylverätherte Methylolmelamin besitzt bei 25 °C eine Viskosität von 9,6 Pa·s und enthält pro Mol Melamin ca. 5,8 Mole Formaldehyd und ca. 4,6 Mole methylverätherte Methylolgruppen.

Sofern in den vorstehenden Beispielen nichts anderes angegeben ist, betrug der Methanolgehalt der verwendeten Formaldehydlösungen weniger als 1 Gew.%.

Ansprüche

1. Verfahren zur Herstellung weitgehend monomerer, mit Methanol verätherter Methylolmelamin, die pro Mol Melamin mehr als 5 Mole Formaldehyd und mehr als 4 Mole methylverätherter Methylolgruppen enthalten und bei einem Festkörpergehalt von mindestens 95 % bei 25 °C eine Viskosität von weniger als 20 Pa·s besitzen, dadurch gekennzeichnet, daß Melamin mit einer wässrigen, zwischen 30 und 50 Gew.% Formaldehyd enthaltenden Formalinlösung, die auf pH-Werte von 6,7 bis 7,3 eingestellt wurde, im Molverhältnis 1 : 6,5 bis 1 : 8 während 1 1/2 bis 5 Stunden bei 50 bis 85 °C methyloliert wird und die erhaltene Suspension des Methylolmelamins mit 15 bis 30 Molen Methanol pro Mol Melamin bei Temperaturen von 30 bis 60 °C in Gegenwart einer starken Säure veräthert wird.

2. Verfahren nach Anspruch 1, dadurch gekennzeichnet, daß Melamin und Formaldehyd im Molverhältnis 1 : 7 bis 1 : 7,5 eingesetzt werden.

3. Verfahren nach den Ansprüchen 1 und 2, dadurch gekennzeichnet, daß der pH-Wert der Formalinlösung mit Borax eingestellt worden ist.

4. Verfahren nach den Ansprüchen 1 bis 3, dadurch gekennzeichnet, daß die Methylolierung bei Temperaturen von 65 bis 75 °C durchgeführt wird.

5. Verfahren nach den Ansprüchen 1 bis 4, dadurch gekennzeichnet, daß die Methylolierung im Verlauf von 2 bis 3 Stunden durchgeführt wird.

6. Verfahren nach den Ansprüchen 1 bis 5, dadurch gekennzeichnet, daß die Verätherung bei Temperaturen von 35 bis 45 °C durchgeführt wird.

7. Verfahren nach den Ansprüchen 1 bis 6, dadurch gekennzeichnet, daß bei der Verätherung als starke Säure Schwefelsäure, Salzsäure, Phosphorsäure, Trichloressigsäure und/oder p-Toluolsulfonsäure verwendet werden.

8. Verfahren nach den Ansprüchen 1 bis 7, dadurch gekennzeichnet, daß bei Verätherung als starke Säure Salpetersäure verwendet wird.

9. Verfahren nach den Ansprüchen 1 bis 8, dadurch gekennzeichnet, daß die bei der Methylolierung erhaltene Suspension von Methylolmelamin im selben Gefäß ohne Isolierung und ohne Zwischentrocknung des Methylolmelamins veräthert wird.

10. Verfahren nach den Ansprüchen 1 bis 9, dadurch gekennzeichnet, daß nach beendeter Methylolierung eine Abkühlung auf Verätherungstemperatur erst nach Zusatz des Methanols erfolgt.

Claims

1. Process for manufacture of methanol-etherified methylolmelamines which are largely monomeric containing more than 5 mols of formaldehyde and more than 4 mols of methyl-etherified methylol groups, per mol of melamine, and having a viscosity of less than 20 pascal-seconds at 25 °C at a solid content of at least 95 % by weight, characterised in that melamine is methylolated with an aqueous solution of formalin containing 30 to 50 % by weight of formaldehyde and the pH of which has been adjusted to 6.7 to 7.3 at a molar ratio of 1 : 6.5 to 1 : 8 for 1 1/2 to 5 hours at 50 °C to 85 °C and the resulting suspension of methylolmelamine is etherified with 15 to 30 mols of methanol per mol of melamine at temperatures of 30 °C to 60 °C in the presence of a strong acid.

2. Process according to claim 1, characterised in that melamine and formaldehyde are employed at a molar ratio of 1 : 7 to 1 : 7.5.

3. Process according to claims 1 and 2, characterised in that the pH of the formalin solution has been adjusted with borax.

4. Process according to claims 1-3, characterised in that methylolation is carried out at temperatures from 65 °C to 75 °C.

5. Process according to claims 1-4, characterised in that methylolation is carried out over a period of 2 to 3 hours.

6. Process according to claims 1-5, characterised in that etherification is carried out at temperatures from 35 °C to 45 °C.

7. Process according to claims 1-6, characterised in that the strong acid used in the etherification is sulphuric acid, hydrochloric acid, phosphoric acid, trichloroacetic acid and/or p-toluenesulphonic acid.

8. Process according to claims 1-7, characterised in that the strong acid used in the etherification is nitric acid.

9. Process according to claims 1-8, characterised in that the suspension of methylolmelamine obtained in the methylolation reaction is etherified in the same vessel without isolating and without intermediately drying the methylolmelamine.

10. Process according to claims 1-9, characterised in that, methylolation having been completed, the mixture is cooled to the etherification temperature only after addition of the ethanol.

**Revendications**

1. Procédé de préparation de méthylol-mélamines éthérifiées par le méthanol, largement monomères, comprenant par mole de mélamine plus de 5 moles de formaldéhyde et plus de 4 moles de groupes méthyloliques éthérifiés par le méthanol et dont la viscosité à 25 °C est inférieure à 20 Pa·s pour une teneur en matière solide d'au moins 95 % en poids, procédé caractérisé en ce que l'on méthylole la mélamine avec une solution aqueuse de formaline à 30-50 % en poids de formaldéhyde ajustée à un pH de 6,7 à 7,3, dans un rapport molaire de 1 : 6,5 à 1 : 8, à une température comprise entre 50 et 85 °C pendant une durée d'une heure et demie à 5 heures, puis, sur la suspension de méthylolmélamine ainsi formée, on effectue l'éthérification avec 15 à 30 moles de méthanol par mole de mélamine à des températures de 30 à 60 °C, en présence d'un acide fort.

2. Procédé selon la revendication 1, caractérisé en ce que l'on fait réagir la mélamine avec le formaldéhyde dans un rapport molaire de 1 : 7 à 1 : 7,5.

3. Procédé selon l'une des revendications 1 et 2, caractérisé en ce que le pH de la solution de formaline est ajusté avec du borax.

4. Procédé selon l'une quelconque des revendications 1 à 3, caractérisé en ce que la méthylolation est effectuée à des températures de 65 à 75 °C.

5. Procédé selon l'une quelconque des revendications 1 à 4, caractérisé en ce que la méthylolation est effectuée en 2 à 3 heures.

6. Procédé selon l'une quelconque des revendications 1 à 5, caractérisé en ce que l'éthérification est effectuée à des températures de 35 à 45 °C.

7. Procédé selon l'une quelconque des revendications 1 à 6, caractérisé en ce que l'on utilise comme acide fort dans l'éthérification l'acide sulfurique, chlorhydrique, phosphorique, trichloracétique et/ou p-toluène-sulfonique.

8. Procédé selon l'une quelconque des revendications 1 à 7, caractérisé en ce que l'on utilise comme acide fort dans l'éthérification l'acide nitrique.

9. Procédé selon l'une quelconque des revendications 1 à 8, caractérisé en ce que la suspension de méthylol-mélamine résultant de la méthylolation est soumise à l'éthérification dans le même récipient réactionnel, sans isolement ni séchage préalable de la méthylol-mélamine.

10. Procédé selon l'une quelconque des revendications 1 à 9, caractérisé en ce que, une fois la méthylolation terminée, on ne refroidit le mélange réactionnel à la température d'éthérification qu'après lui avoir ajouté le méthanol.